# EUROPÄISCHE PATENTSCHRIFT

(11) **EP 1 347 768 B1**
(45) Veröffentlichungstag und Bekanntmachung des Hinweises auf die Patenterteilung: **23.01.2008**
(21) Anmeldenummer: 01989530.9
(22) Anmeldetag: 28.11.2001
(51) Int. Cl.: A61K 36/00, A61P 17/00

(54) **KOSMETISCHE UND/ODER DERMOPHARMAZEUTISCHE ZUBEREITUNGEN ENTHALTEND EXTRAKTE AUS DEN BLÄTTERN DER PFLANZE ARGANIA SPINOSA**
COSMETIC AND/OR DERMOPHARMACEUTICAL PREPARATIONS CONTAINING LEAF EXTRACTS OF THE PLANT ARGANIA SPINOSA
PREPARATIONS COSMETIQUES ET/OU DERMOPHARMACEUTIQUES CONTENANT DES EXTRAITS DE FEUILLES DE LA PLANTE I ARGANIA SPINOSA /I

(30) Priorität: 06.12.2000 EP 00440319
(43) Veröffentlichungstag der Anmeldung: 01.10.2003
(73) Patentinhaber: Cognis France, S.A.S., 31360 Boussens (FR)
(72) Erfinder: PAULY, Gilles, F-54000 Nancy (FR); HENRY, Florence, F-54600 Villers-les-Nancy (FR); DANOUX, Louis, F-54420 Saulxures Les Nancy (FR); CHARROUF, Zoubida, B.P. 1014 Rabat R.P. (MA)
(74) Vertreter: Fabry, Bernd
(86) Internationale Anmeldenummer: PCT/EP2001/013885
(87) Internationale Veröffentlichungsnummer: WO 2002/045728

(56) Entgegenhaltungen:
- EP-A- 0 496 649
- EP-A- 0 545 147
- EP-A- 0 768 079
- WO-A-00/69404
- WO-A-98/19651
- US-A- 5 211 944
- TAHROUCH, SAIDA (1) ET AL: "Polyphenol investigation of Argania spinosa (Sapotaceae) endemic tree from Morocco." ACTA BOTANICA GALLICA, (2000) VOL. 147, NO. 3, PP. 225-232. PRINT., XP000996078
- DATABASE WPI Section Ch, Week 199732 Derwent Publications Ltd., London, GB; Class D21, AN 1997-349222 XP002165748 & NZ 264 108 A (COMVITA NZ LTD), 26. Mai 1997 (1997-05-26)
- DATABASE WPI Section Ch, Week 199344 Derwent Publications Ltd., London, GB; Class B04, AN 1993-348331 XP002165749 & JP 05 255060 A (EIKODO KK), 5. Oktober 1993 (1993-10-05)
- PATENT ABSTRACTS OF JAPAN vol. 1998, no. 13, 30. November 1998 (1998-11-30) & JP 10 218780 A (PANACEA BIOTEC LTD;UNIV INST OF PHARMACEUT SCI), 18. August 1998 (1998-08-18)

## Beschreibung

### Gebiet der Erfindung

Die Erfindung befindet sich auf dem Gebiet der Pflegestoffe und betrifft Zubereitungen enthaltend Extrakte aus den Blättern der Pflanze Argania spinosa sowie Verwendung von Extrakten aus den Blättern der Pflanze Argania spinosa als neue Haut - und Haarpflegemittel.

### Stand der Technik

Kosmetische Zubereitungen stehen dem Verbraucher heute in einer Vielzahl von Kombinationen zur Verfügung. Dabei wird nicht nur erwartet, dass diese Kosmetika einen bestimmten pflegenden Effekt zeigen oder einen bestimmten Mangel beheben, sondern immer häufiger wird nach Produkten verlangt, die mehrere Eigenschaften gleichzeitig aufweisen und somit ein verbessertes Leistungsspektrum zeigen. Von besonderem Interesse sind Stoffe, die sowohl Wirkstoffe darstellen, die für Haut und/oder Haare beispielsweise pflegende, vor Alterserscheinungen schützende, revitalisierende Eigenschaften vermitteln als auch gleichzeitig die technischen Eigenschaften des kosmetischen Produktes, wie Lagerstabilität, Lichtstabilität und Formulierbarkeit positiv beeinflussen oder zumindest nicht verschlechtern. Hierbei sind zusätzlich eine gute Hautverträglichkeit und besonders der Einsatz natürlicher Produkte beim Kunden gefragt. Daneben ist es wünschenswert, durch Kombination bereits bekannter Wirkstoffe, oder durch auffinden neuer Einsatzgebiete bereits bekannter Substanzklassen deutlich bessere Produkte zu erhalten. Ein Nachteil besteht hier allerdings häufig darin, das eine Kombination von Wirkstoffen erst dann erhalten wird, wenn unterschiedliche Pflanzenextrakte gleichzeitig in unterschiedlichen Mengenverhältnissen verwendet werden.

Extrakte von Pflanzen und deren Inhaltstoffe finden immer häufiger Einsatz in der Kosmetik und Pharmazie. Pflanzenextrakte werden seit vielen Jahren in den unterschiedlichsten Kulturen für medizinische aber auch bereits für kosmetische Zwecke genutzt. Oftmals waren für diese Pflanzenextrakte nur ganz bestimmte einzelne Wirkungen bekannt und das Einsatzgebiet sehr eingeschränkt.

WO 01/82885 offenbart kosmetische oder pharmazeutische Zubereitungen, weiche Saponine enthalten. Die Saponine stammen dabei aus einem Extrakt der Pflanze Argania spinosa.

### Beschreibung der Erfindung

Die Aufgabe der vorliegenden Patentanmeldung hat darin bestanden, kosmetische und/oder dermopharmazeutische Zubereitungen zur Verfügung zu stellen, die einen Einsatz in der Kosmetik oder auch der Pharmazie ermöglichen und neben pflegenden Eigenschaften vor allem verbesserte schützende Eigenschaften für menschliche Haut und/oder Haare beispielsweise gegen UV-Strahlung und anderen Umwelteinflüssen besitzen und gleichzeitig vorbeugende und heilende Wirkung bei Alters-erscheinungen der Haut zeigen, die Melanogenese beeinflussen können und antiinflammatorisch einsetzbar sind.

Eine weitere Aufgabe der vorliegenden Patentanmeldung hat darin bestanden, Zubereitungen zur Verfügung zu stellen, die Wirkstoffe aus nachwachsenden Rohstoffen enthalten und gleichzeitig vielseitig als Pflegemittel in der Kosmetik sowohl in der Hautkosmetik, als auch in der Haarpflege einsetzbar sind.

Gegenstand der Erfindung sind Zubereitungen, die Extrakte aus den Blättern der Pflanze Argania spinosa enthalten als Pflegemittel für Haut und Haare.

Überraschenderweise wurde gefunden, dass durch den Einsatz von Extrakten aus den Blättern der Pflanze Argania spinosa Produkte erhalten werden, die gleichzeitig gute pflegende und schützende Eigenschaften für Haut und Haar aufweisen, sowie eine hohe Hautverträglichkeit besitzen. Die so erhaltenen Mittel zeichnen sich durch besonders gute Effekte in der Hautkosmetik aus. Sie zeigen neben schützenden Effekten auch eine vorbeugende und heilende Wirkung bei Alterserscheinungen der Haut. Sie beeinflussen die Melanogenese und zeigen eine anti-inflammatorische und antimikrobielle Aktivität.

Diese mehrfachen Einsatzgebiete der erfindungsgemäßen Mittel aus dem nachwachsendem Rohstoff der Pflanze Argania spinosa macht es für den Markt und für den Verbraucher sehr attraktiv. Die komplexe Aufgabe der Erfindung konnte somit durch den Einsatz von Extrakten aus den Blättern der Pflanze Argania spinosa gelöst werden.

Der Begriff Zubereitungen wird im Rahmen der Erfindung synonym mit dem Begriff Mittel oder Pflegemittel verwendet.

### Argania spinosa

Die erfindungsgemäß einzusetzenden Extrakte werden aus den Blättern einer Pflanze der Familie der Sapotaceae, speziell aus Argania spinosa gewonnen. Bei dieser Pflanze handelt es sich um einen an den Ölbaum erinnernden Baum, der überwiegend in Marokko an der Westseite des Atlasgebirges zu finden ist. Er bildet an seinen knorrigen Ästen und bedornten Zweigen Beeren von der Größe und Gestalt der Oliven mit ein bis zwei Samenkernen. Das nussartig schmeckende Öl aus den Samenkernen dient unter anderem als Speiseöl.

### Extraktion

Die Herstellung der erfindungsgemäß einzusetzenden Extrakte erfolgt durch übliche Methoden der Extraktion der Blätter der Pflanzen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S. 1026-1030, Springer Verlag, Berlin-Heidelberg-New-York 1991) verwiesen. Als Ausgangsmaterial können frische oder getrocknete Blätter der Pflanzen eingesetzt werden, üblicherweise wird jedoch von Blättern der Pflanzen ausgegangen, die vor der Extraktion mechanisch zerkleinert werden können. Hierbei eignen sich alle dem Fachmann bekannten Zerkleinerungsmethoden, als Beispiel sei die Zerkleinerung mit einem Klingen enthaltenen Gerät genannt.

Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ether, Ketone oder halogenhaltige Kohlenwasserstoffe mit mehr oder weniger hohen Wassergehalten (destilliert oder nicht destilliert) vorzugsweise wässrig, alkoholische Lösungen mit mehr oder weniger hohen Wassergehalten, verwendet werden. Besonders bevorzugt ist die Extraktion mit Wasser, Methanol, Ethanol, Propanol, Butanol und deren Isomere, Aceton, Propylenglycolen, Polyethylenglycolen Ethylacetat, Dichlormethan, Trichlormethan sowie Mischungen hieraus. Die Extraktion erfolgt in der Regel bei 20 bis 100 °C, bevorzugt bei 80 bis 100°C, insbesondere bei Siedetemperatur der Lösungsmittel oder Lösungsmittelgemische. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt.

Die vorliegende Erfindung umfasst die Erkenntnis, dass die Extraktionsbedingungen sowie die Ausbeuten der Endextrakte je nach gewünschtem Einsatzgebiet gewählt werden können.

Die Einsatzmenge der Pflanzenextrakte in den genannten Zubereitungen richtet sich nach der Konzentration der einzelnen Inhaltstoffe und nach der Art der Anwendungen der Extrakte. Die Gesamtmenge des Pflanzenextraktes, der in den erfindungsgemäßen Zubereitungen enthalten ist, beträgt in der Regel 0,01 bis 25 Gew.-%, vorzugsweise 0,03 bis 5 Gew.%, insbesondere 0,03 bis 0,6 Gew.% berechnet als Trockengewicht, bezogen auf die Zubereitungen, mit der Maßgabe, dass sich die Mengenangaben mit Wasser und gegebenenfalls weiteren Hilfs- und Zusatzstoffen zu 100 Gew.-% addieren.

Der Gesamtanteil der Hilfs- und Zusatzstoffe kann 1 bis 50, vorzugsweise 5 bis 40 Gew.-% - bezogen auf die kosmetischen und/oder dermopharmazeutischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

### Extrakte

Die erfindungsgemäßen Extrakte aus den Blättern der Pflanze Argania spinosa enthalten in der Regel als Wirkstoffe Flavonderivaten, Saponoside, Oligomere Procyanolidine und Sterole. Diese sind je nach gewähltem Ausgangsmaterial und nach gewählter Extraktionsmethode unterschiedlich zusammengesetzt. Eine besondere Ausführungsform der Erfindung sind daher kosmetische und/oder dermopharmazeutische Zubereitungen, die Extrakte aus den Blättern von Argania spinosa enthalten, die Substanzen enthalten, die ausgewählt sind aus der Gruppe, die gebildet wird von Flavonderivaten, Saponoside, Oligomere Procyanolidine und Sterole.

Im Sinne der vorliegenden Erfindung sind unter Flavonderivaten solche zu verstehen, die sich aus den Blättern der Pflanze Argania spinosa isolieren lassen. Im Besonderen handelt es sich um Stoffe, die Hydrierungs-, Oxidations- oder Substitutionsprodukte des 2-Phenyl-4H-1-benzopyrans darstellen, wobei eine Hydrierung in der 2,3-Stellung des Kohlenstoffgerüsts bereits vorliegen kann, eine Oxidation in der 4-Stellung bereits vorliegen kann, und unter Substitutionsprodukte der Ersatz eines oder mehrerer Wasserstoffatome durch Hydroxy- oder Methoxy-Gruppen zu verstehen ist. Bei dieser Definition sind also Flavane, Flavan-3-ole (Catechine), Flavan-3,4-diole (Leukoanthocyanidine), Flavone, Flavonole und Flavanone im herkömmlichen Sinn eingeschlossen. In einer besonderen Ausführungsform der Erfindung handelt es sich um glycosidierte Flavonderivate, insbesondere um Myricetinglycosid, Quercetinglycosid, Gossypetinglycosid, Kämpferolglycosid und Luteolinglycosid.

Unter Saponosiden sind im Sinne der Erfindung solche zu verstehen, die sich aus den Blättern der Pflanze Argania spinosa isolieren lassen. Im Besonderen handelt es sich um eine Gruppe von Glykosiden, die in Wasser kolloidale, seifenartige Lösungen bilden.

Unter Sterolen sind im Sinne der Erfindung Steroide zu verstehen, die sich aus den Blättern der Pflanze Argania spinosa isolieren lassen. Im Besonderen handelt es sich um Spinasterol (Δ7 Sterol), Scottenol und/oder Steroide, die nur an C-3 eine Hydroxy-Gruppe, sonst aber keine funktionelle Gruppe tragen, also formal Alkohole darstellen. Zusätzlich besitzen die 27 bis 30 C-Atome enthaltenden Sterole im allgemeinen eine C=C-Doppelbindung in 5/6-Stellung, seltener auch/oder in 7/8, 8/9 und anderen Positionen (z. B. 22/23).

Unter Oligomeren Procyanolidinen (OPC) versteht man im Sinne der Erfindung solche, die aus den Blättern der Pflanze Argania spinosa isoliert werden können.

Sie enthalten als Monomerbausteine die im Pflanzenreich weit verbreiteten Tannine. Chemisch betrachtet können zwei Typen von Tanninen unterschieden werden, nämlich kondensierte Formen zu denen auch das Procyanidin A2 gehört, und hydrolysierbare Tannine. Kondensierte Tannine, die auch als Flavolane bezeichnet werden, entstehen in der Biosynthese durch Kondensation von Monomeren, wie z.B. Catechin, Gallocatechin, Afzelechin (2-R, 3-S Typ Monomere) sowie Epicatechin, Epigallocatechin und Epiafzelechin (2-R, 3-R Typ Monomere). Durch Kondensation der Monomeren entstehen zunächst Dimere und dann höhere Oligomere, wobei die Kondensation durch Ausbildung einer C-C-Bindung in 4-8 bzw. 6-8-Position erfolgt. Im Fall der bevorzugten A2-Dimere vom Typ des Proanthocyanidin A2 gibt es eine doppelte Bindung, nämlich C2->O->C7 und C4->C8. Die Struktur ist in der folgenden Abbildung wiedergegeben:

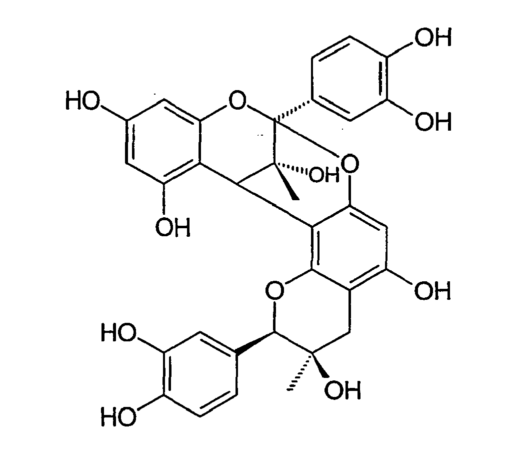

Die A2-Typ Proanthocyanidine sind weniger hydrolyseanfällig als die B-Typen. Im übrigen wird dieser Begriff synonym für die Gruppe der kondensierten Tannine verwendet, da diese unter dem Einfluss heißer Mineralsäuren Monomere abspalten.

Zur Erhöhung der Stabilität in Formulierungen werden die OPC vorzugsweise nach Extraktion derivatisiert und die erhaltenen Derivate in den Formulierungen eingesetzt. Als besonders bevorzugt gelten hier die Ester mit OPC.

Ein weiterer Gegenstand der Erfindung ist die Verwendung von Extrakten aus den Blättern der Pflanze Argania spinosa als Pflegemittel für die Haut und/oder die Haare. Diese Art der Verwendung umfasst sowohl Mittel mit kosmetischer als auch mit dermopharmazeutischer Wirkung.

### Pflegemittel:

Als Pflegemittel im Sinne der Erfindung sind Pflegemittel für Haut und Haar zu verstehen. Diese Pflegemittel schließen unter anderem reinigende und aufbauende Wirkung für Haut und Haare ein.

Die Applikation kann sowohl topisch als auch oral in Form von Tabletten, Dragees, Kapseln, Säfte, Lösungen und Granulate erfolgen.

Die erfindungsgemäßen Zubereitungen zeigen darüber hinaus eine hervorragende hautpflegende Wirkung bei gleichzeitig hoher Hautverträglichkeit. Außerdem zeigen sie eine gute Stabilität, insbesondere gegenüber oxidativer Zersetzung der Produkte. Die Zubereitungen weisen eine Vielzahl von kosmetischen und dermopharmazeutischen Wirkungen auf. Weitere Gegenstände der Erfindung betreffen daher die Verwendung von Extrakten aus den Blättern der Pflanze Argania spinosa
➢ als Sonnenschutzmittel; insbesondere gegen UVA-Strahlung und/oder gegen UVB-Strahlung;
➢ als Antioxidant;
➢ als anti-inflammatorische Mittel
➢ als anti-mikrobielle Mittel
➢ als Mittel gegen die Hautalterung
➢ als protease-inhibierendes Mittel, insbesondere als MMP- und/oder Collagenase- und/oder Elastase-inhibierendes Mittel und bevorzugt als Plasmin Inhibitoren;
➢ als Pigmentierungsmittel.

### Sonnenschutzmittel bzw. UV-Lichtschutzfaktoren

Die Extrakte aus den Blättern der Pflanze Argania spinosa wirken im Sinne der Erfindung als Sonnenschutzmittel.

Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) u. UV-A (315-400 nm).

Die Pigmentierung normaler Haut unter dem Einfluss der Sonnenstrahlung, d. h. die Bildung von Melaninen, wird durch UV-B u. UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Körnern. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) u. gar Brandblasen führen kann.

Als UV-Absorber oder Lichtfilter, die also die UV-Strahlung in unschädliche Wärme umwandeln, werden Extrakte aus den Blättern der Pflanze Argania spinosa eingesetzt, diese können zusätzlich in Kombination mit weiteren Sonnenschutzmitteln bzw. UV-Lichtschutzfaktoren vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP-0693471-B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

### Als wasserlösliche Substanzen kommen in Frage:

➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bomylidenmethyl)benzolsulfonsäure und 2-Methyl-5-(2-oxo-3-bornyliden)sulfonsäure und deren Salze.

Als typische UVA-Filter kommen insbesondere Derivate des Benzoylmethans in Frage, wie beispiels-weise 1-(4'-tert.Butylphenyl)-3-(4'-methoxyphenyl)propan-1,3-dion, 4-tert.-Butyl-4'-methoxydibenzoyl-methan (Parsol 1789), 1-Phenyl-3-(4'-isopropylphenyl)-propan-1,3-dion sowie Enaminverbindungen, wie beispielsweise beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titand0ioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parfümerie und Kosmetik 3 (1999), Seite 11ff zu entnehmen.

Die Extrakte aus den Blättern der Pflanze Argania spinosa wirken im Sinne der Erfindung gegen die Schädigung von Fibroblasten und/oder Keratinocyten durch UVA-Strahlung und/oder UVB-Strahlung. UVA-Strahlen dringen bis in die Dermis ein, wo sie zu Oxidationsstreß führen, was durch eine Lipoperoxidation der Zytoplasmamembranen nachgewiesen wird. Die Lipoperoxide werden zu Malonaldialdehyd (MDA) abgebaut, der viele biologische Moleküle wie Proteine und Nukleinbasen vernetzen wird (Enzymhemmung bzw. Mutagenese). Die erfindungsgemäßen Extrakte der Pflanze Argania spinosa reduzieren signifikant den Grad an MDA in humanen Fibroblasten, welcher durch UVA-Strahlen induziert wird und zeigen damit eine hohe Kapazität schädliche Effekte eines oxidativen Stresses auf der Haut zu reduzieren.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Der Grad der Freisetzung des Cytoplasaenzyms LDH (Lactat Dehydrogenase) in humanen Keratinocyten dient als Marker für eine Zellschädigung.

Die erfindungsgemäßen Extrakte aus den Blättern der Pflanze Argania spinosa reduzieren den Effekt von UVB-Strahlung auf die Anzahl an Keratinocyten und auf den Gehalt an freigesetzte LDH. Die Extrakte zeigen demnach die Fähigkeit, die durch UVB-Strahlung hervorgerufene Schädigung an Zellmembranen zu reduzieren.

Die Extrakte aus den Blättern der Pflanze Argania spinosa wirken im Sinne der Erfindung als Antioxidant bzw Radikalfänger.

Als Antioxidantien im Sinne der Erfindung sind Oxidationsinhibitoren zu verstehen, die sich aus den Blättern der Pflanze Argania spinosa isolieren lassen. Antioxidantien sind in der Lage, die unerwünschten, durch Sauerstoff-Einwirkungen und andere oxidative Prozesse bedingten Veränderungen in den zu schützenden Stoffen zu hemmen oder zu verhindern. Die Wirkung der Antioxidantien besteht meist darin, dass sie als Radikalfänger für die bei der Autoxidation auftretenden freien Radikale wirken.

Neben der Verwendung von Extrakten der Pflanze Argania spinosa als Antioxidantien können auch weitere, bereite bekannte Antioxidantien eingesetzt werden. Eine mögliche Anwendung der Antioxidantien zum Beispiel in kosmetischen und/oder dermopharmazeutischen Zubereitungen, ist die Anwendung als sekundäre Lichtschutzmittel, weil Antioxidantien in der Lage sind, die photochemische Reaktionskette zu unterbrechen, welche ausgelöst wird, wenn UV-Strahlung in die Haut eindringt. Neben dem erfindungsgemäßen Pflanzenextrakt sind weitere typische Beispiele hierfür Aminosäuren (z.B. Glycin, Alanin, Arginin, Serin, Threonin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin, Lutein) oder deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, -Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmifinsäure, Phytinsäure, Lactoferrin), α-Hydroxysäuren (z.B: Citronensäure, Milchsäure, Äpfelsäure), Huminsäure, Gallensäure, Gallenextrakte, Bilirubin, Biliverdin, Boldin, Boldo-Extrakt, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄ Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die weiteren UV-Lichtschutzfaktoren bzw. Antioxidantien können in Mengen von 0,01 bis 25, vorzugsweise 0,03 bis 10 und insbesondere 0,1 bis 5 Gew.-% bezogen auf die Gesamtmenge in den Zubereitungen, zugegeben werden.

Die Extrakte aus den Blättern der Pflanze Argania spinosa wirken im Sinne der Erfindung als antiinflammatorisches Pflegemittel, die eine Entzündung der Haut heilen können oder die einer Entzündung vorbeugen können. Die Entzündungen können dabei die unterschiedlichsten Ursachen aufweisen. Insbesondere können Entzündungen behandelt werden, die durch UV-Strahlung, Hautverunreinigungen oder bakteriell wie hormonell bedingte Hautveränderungen, z. B. Akne induziert werden.

Die Extrakte aus den Blättern der Pflanze Argania spinosa wirken im Sinne der Erfindung als anti-mikrobielle Mittel, insbesondere gegen jede Art der bakteriell bedingten Hautveränderung. Diese Art der Hautveränderung schließt die Infektion durch Bakterien der unterschiedlichsten Arten und Gattungen mit ein, wie beispielsweise Staphylokokken, Streptokokken, Streptomyceten und/oder Propionebakterien.

Die Extrakte aus den Blättern der Pflanze Argania spinosa wirken im Sinne der Erfindung gegen Hautalterungen, insbesondere gegen jede Art der Fältchen- und Faltenbildung. Eine andere Bezeichnung für diese Art der Pflegemittel ist auch anti-ageing Mittel. Die Verwendungen schließen eine Verlangsamung von Altersprozessen der Haut mit ein. Die Alterserscheinungen können die unterschiedlichsten Ursachen aufweisen. Insbesondere können diese Alterserscheinungen auf Grund von Apoptose, durch UV-Strahlung oder durch die Zerstörung der hauteigenen Proteine wie beispielsweise Collagen oder Elastan induzierten Schädigungen der Haut verursacht sein.

Die erfindungsgemäßen Extrakte aus Extrakten aus den Blättern der Pflanze Argania spinosa wirken als Protease-inhibierendes Mittel, insbesondere als MMP- und/oder Collagenase- und/oder Elastase-inhibierendes Mittel und bevorzugt als Plasmin Inhibitoren. Unter MMP versteht man Matrix-Metallo-Proteasen. Zu den Matrix-Metallo-Proteasen zählen u.a. Collagenase, aber auch eine bestimmte Art der Elastasen. Die Aktivität der Enzyme ist abhängig von Metallionen - häufig handelt es sich um Zn²+-lonen. Die hauptsächlich vorkommende Elastase zählt zur Gruppe der Serin-Proteasen. Ihre katalytische Reaktion beruht auf einen anderen Mechanismus. Diese Proteasen (Collagenase und die unterschiedlichen Elastasen) katalysieren die Fragmentierung und Zerstörung der dermalen Makromoleküle wie Proteoglycan, Collagen und Elastin und führen dadurch zur Alterung der Haut und zu den Effekten der natürlichen Hautalterung nach UV-Strahlung.

Bei inflammatorischen Prozessen in der Haut werden von den Makrophagen und von polymorphonuclearen neutrophilen Granulocyten Proteasen wie z.B. die Serin-Protease Elastase oder Matrix-Metallo-Proteasen (MMP) wie Collagenase und eine weitere, zu den MMP gehörende Elastin abbauende Elastase ausgeschüttet. Des weiteren werden bei älteren Menschen oder nach UV-Strahlung durch die dermalen Fibroblasten interstitial Collagenasen - oder auch MMP-1 genannt - ausgeschüttet.

Im menschlichen Gewebe finden sich Inhibitoren dieser Matrix-Metallo-Proteasen, deren Bildung und Konzentration mit dem Alter abnimmt. Ihre Bezeichnung wird mit TIMP (Tissue-inhibitor of metalloprotease) abgekürzt. Die erfindungsgemäßen Extrakte sind in der Lage die Bildung dieser natürlich vorkommenden Inhibitoren zu stimulieren.

Neben den bereits genannten Effekten der Extrakte aus den Blättern der Pflanze Argania spinosa wurden positive Effekte gefunden bei der Beeinflussung der Melanogenese. Die Melanogenese bezeichnet die natürliche Synthese von Melanin in den Zellen, speziell den Melanozyten. Diese natürliche Pigmentierung-kann beeinflusst- werden, indem in die Reaktionskette der Oxidation von Tyrosin über L-DOPA bis zum Melanin eingegriffen wird. Hautaufhellenden Effekten erzielt man durch die Inhibierung der Melanogenese, während eine Stimulierung der Melanogenese zur erhöhten Pigmentierung führen kann. Die wässrig/alkoholische Extrakte aus den Blättern der Pflanze Argania spinosa, insbesondere wässrig/ethanolische Extrakte, zeigen eine Stimulierung der Melanogenese. Diese Effekte erlauben den Einsatz als Pigmentierungsmittel bzw. als Selbstbräuner.

Neben den unterschiedlichen Extrakten aus den Blättern der Pflanze Argania spinosa können die Zubereitungen weitere Selbstbräuner oder Tyrosinaseinhibitoren enthalten. Als Selbstbräuner eignet sich Dihydroxyaceton. Als Tyrosinaseinhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Die Verwendung der erfindungsgemäßen Extrakte als schützende und aufbauende Pflegemittel ist prinzipiell für alle Zubereitungen möglich, die zur Prävention gegen Schädigungen oder bei Schädigungen der Haut und/oder Haare und damit in der Haut- und Haarpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Die erfindungsgemäßen Zubereitungen können zur Herstellung von kosmetischen und/oder dermopharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben zum Einsatz kommen. Des weiteren können die erfindungsgemäßen Zubereitungen zur oralen Applikation auch in Tabletten, Dragees, Kapseln, Säfte, Lösungen und Granulate eingearbeitet sein.

Diese Zubereitungen können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, Deodorantien, Antitranspirantien, Antischuppenmittel, Filmbildner, Quellmittel, Insektenrepellentien, Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycefinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 **oder** J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Feftalkoholen bzw. Ester von verzweigten C₆-C₁₃-Carbonsäuren mit linearen oder verzweigten C₆-C₂₂-Fettatkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleyistearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere. Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt. Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsarad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, lsostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioteat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Potygiycerin-3-Diisostearate (Lameform® TGI), Polyglyceryl-4 Isostearate (Isolan® Gl 34), Polyglyceryl-3 Oleate, Diisostearoyl Polyglyceryl-3 Düsostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010190), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®ISandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidonl Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm. Toil. 108, 95 (1993**)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### Biogene Wirkstoffe

Unter biogenen Wirkstoffen sind im Rahmen der Erfindung zusätzlich solche zu verstehen, die nicht aus der Pflanze Argania spinosa stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, weitere Pflanzenextrakte und zusätzliche Vitaminkomplexe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 - dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise-Citronensäure, Äpfelsäure , Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen.

Wässrige oder wasserfreie Formulierungen von Antitranspirantien enthalten typischerweise folgende Inhaltsstoffe:
➢ adstringierende Wirkstoffe,
➢ Ölkomponenten;
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsaure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.%, bezogen auf die gesamte Mischung, eingesetzt.

### Beispiele

### 1. Beispiel: Extraktion der Pflanzen mit destilliertem Wasser

0,3 kg zerkleinerte Blätter der Pflanze Argania spinosa wurden in ein Glasgefäß überführt und mit 3 destilliertem Wasser aufgegossen. Die Mischung wurde bei 80-90 °C für eine Stunde gerührt. Anschließend ließ man die Mischung auf Raumtemperatur abkühlen und zentrifugierte 15 min bei einer Geschwindigkeit von 5000 g. Die überstehende kolloide Flüssigkeit wurde durch Filtration an Tiefenfilter mit einer mittleren Porosität von 450 nm (von der Firma Seitz, Bordeaux Frankreich) vom Rückstand getrennt und anschließend sprühgetrocknet. Die Ausbeute Trockenprodukt berechnet auf Trockengewicht der eingesetzten Blätter betrug 25,6 Gew.-%.

### 2. Beispiel: Extraktion der Pflanzen mit wässrigem Methanol

Beispiel 1 wurde wiederholt, die Extraktion jedoch mit 1180 Gew.-%-igem wässrigen Methanol und 0,1 kg zerkleinerte Blätter durchgeführt. Die Extraktion wurde unter Rühren 1 h bei Siedetemperatur unter reflux durchgeführt und der Extrakt wie beschrieben weiter verarbeitet. Die Filtration wurde wie im Beispiel 1 beschrieben durchgeführt, jedoch wurde der Rückstand erneut mit 100 ml 80 Gew.%-igem wässrigen Methanol gewaschen. Anschließend wurde zunächst der Alkohol bei 30 °C unter vermindertem Druck entfernt und dann der Rückstand wie beschrieben sprühgetrocknet. Die Ausbeute an Trockenprodukt betrug 28,0 Gew.-% berechnet auf das Trockengewicht an eingesetzten Pflanzen.

### 3. Beispiel: Extraktion der Pflanzen mit wässrigem Ethanol

Beispiel 1 wurde wiederholt, die Extraktion jedoch mit 31 wässrigen Ethanol und 0,1 kg Blätter durchgeführt, wobei das Volumenverhältnis von Ethanol zu Wasser 6 zu 4 betrug. Die Extraktion wurde unter Rühren 1 h bei Siedetemperatur unter reflux durchgeführt und der Extrakt wie beschrieben weiter verarbeitet. Die Filtration wurde wie im Beispiel 1 beschrieben durchgeführt und der Rückstand nochmals mit 0,30 1 Ethanol gewaschen. Anschließend wurde zunächst der Alkohol bei 30 °C unter vermindertem Druck entfernt und dann der Rückstand sprühgetrocknet. Die Ausbeute an Trockenprodukt betrug 21,53 Gew.-% bezogen auf das Trockengewicht an eingesetzten Pflanzen.

### 4. Beispiel: Antimikrobielle Wirksamkeit

Zur Bestimmung der antimikrobiellen Wirksamkeit wurden 6 mm große Plättchen aus Filterpapier, welche mit 20 µl verschiedener Testlösungen (1 % und 5 %) getränkt waren, aufgebracht auf die Oberfläche einer frisch mit *Staphylococcus aureus* versetzten Agar-Zubereitung (1,5 10⁶ Bakterien/ml). Zur Herstellung dieser Agar-Zubereitung wurde eine Agar-Lösung mit 2-4 ml Inokulum suspendiert, in eine Petri-Schale gegeben und 20 min. bei 37 °C getrocknet. Das Inokulum erhielt man durch 18-stündige anaerobe Inkubation der *Staphylococcus aureus* Bakterien. Die Wirksamkeit wurde durch Bestimmung des mittleren Durchmessers der Flächen untersucht, innerhalb derer kein Bakterienwachstum festgestellt werden konnte.

Die Ergebnisse sind in Tabelle 1 zusammengefaßt:

**Tabelle 1: Wirksamkeit gegen Bakterien (Angaben als Durchmesser Inhbierungszone in mm)**

| **Konzentration [Gew.-%]** | **Extrakt, Beispiel 1** | **Extrakt, Beispiel 2** | **Extrakt, Beispiel 3** |
|---|---|---|---|
| 1 | | | 7 |
| 5 | 14 | 12 | 7 |

Die Inhibierungszonen von 7 bis 14 mm zeigen eine deutliche Inhibierung des Wachstums von *Staphylococcus aureus* Bakterien in der Umgebung der mit den Extrakten getränkten Filterplättchen.

### 5. Beispiel: Aktivität gegenüber freien Radikalen

In einer ersten Testreihe wurde die Eignung der Extrakte gegen oxidativen Stress untersucht. Eingesetzt wurden die Extrakte gemäß der Beispiele 1 bis 3 jeweils in einer Konzentration von 0,3 Gew.-%. Als erstes Testsubstrat wurde Diphenylpicrylhydrazyl (DPPH) gewählt, ein purpurrot gefärbtes stabiles Radikal, welches durch Inkontaktbringen mit Radikalfängern in sein ungefärbtes Leucoderivat übergeht. Der Farbwechsel kann photometrisch verfolgt werden. Die Meßergebnisse sind in Tabelle 2 zusammengefaßt ("DPPH-Test"), angegeben ist die Inhibierung von DPPH in %-absolut. In einem weiteren Test wurde als Referenzsystem die Hydroxylierung von Salicylsäure durch Hydroxylradikale (aus der Reaktion von Wasserstoffperoxid mit Eisen(III)ionen und EDTA) untersucht. Auch diese Reaktion kann photometrisch untersucht werden, da das Hydroxylierungsprodukt rötlich gefärbt ist. Gemessen wurde der Einfluß der Extrakte auf die Bildung der Hydroxysalicylsäure bei einer optischen Dichte von 490 nm. Die Meßergebnisse sind ebenfalls in Tabelle 1 zusammengefaßt, angegeben ist wieder die Inhibierung in %-absolut ("Salicylsäure-Test"). In einem dritten und letzten Test wurde Xanthin Oxidase als Testsystem gewählt. Das Enzym bewirkt bei oxidativern Stress die Umwandlung von Purinbasen, wie z.B. Adenin oder Guanin in Uronsäure, wobei die intermediär gebildeten Sauerstoffradikale durch Reaktion mit Luminol über die Lumineszenz nachgewiesen und quantitativ bestimmt werden können. In Gegenwart von Substanzen mit radikalfangenden Eigenschaften vermindert sich die Lumineszenzausbeute. Auch diese Ergebnisse sind in Tabelle 2 zusammengefaßt; wieder ist die Inhibierung in %-absolut angegeben ("Luminol-Test").

**Tabelle 2: Radikalinhibierung [%-absolut]**

| | **DPHH Test** | **Salicylsäure-Test** | **Luminol-Test** |
|---|---|---|---|
| Extrakt nach Beipsiel 1 | 83 | 62 | 100 |
| Extrakt nach Beispiel 2 | 100 | 65 | 100 |
| Extrakt nach Beispiel 3 | 88 | 57 | 100 |

Die Extrakte der Blätter von Argania spinosa zeigten ein hohes Potential freie Radikale und reaktiven Sauerstoff abzufangen und können aus dem Grund hervorragend als Antioxidantien in kosmetischen oder dermopharmazeutischen Zubereitungen eingesetzt werden.

### 6. Beispiel: Zellschutzwirkung gegen UVA an in vitro gezüchteten menschlichen Fibroblasten

Hintergrund: UVA-Strahlen dringen bis in die Dermis ein, wo sie zu Oxidationsstreß führen, was durch eine Lipoperoxidation der Zytoplasmamembranen nachgewiesen wird.

Die Lipoperoxide werden zu Malonaldialdehyd abgebaut, der viele biologische Moleküle wie Proteine und Nukleinbasen vernetzen wird (Enzymhemmung bzw. Mutagenese).

Glutathione (GSH) ist ein Peptid, welches direkt von den Zellen produziert wird um oxidativem Stress oder schädigenden Umwelteinflüssen wie zum Beispiel einer erhöhten Quecksilber- oder Bleibelastung entgegen zu wirken. Der Gehalt an GSH wurde bestimmt nach der Methode von Hissin, beschrieben in Anal. Biochem., 74, 214-226,1976.

Methode: Zur Durchführung dieser Tests wurde ein definiertes Kulturmedium (DMEM) mit 10 % fötalem Kälberserum mit den Fibroblasten beimpft und der Pflanzenextrakt (in dem definierten Medium mit 2% Serum) 72 Stunden nach dem Beimpfen zugegeben.

Nach 48 stündiger Inkubation bei 37 °C und einem CO₂-Gehalt von 5 % wurde das Kulturmedium durch eine Kochsalzlösung ersetzt und die Fibroblasten wurden mit einer UVA-Dosis bestrahlt (20 J/cm²; Röhren: MAZDA FLUOR TFWN40).

Nach der Beendigung der Bestrahlung wurde der Gehalt an Zellproteinen und der Anteil an GSH bestimmt sowie der MDA-Spiegel (Malonaldialdehyd-Spiegel) in der überstehenden Saline-Lösung quantitativ durch Reaktion mit Thiobarbitursäure bestimmt. Die Ergebnisse sind angegeben in Prozent im Vergleich zur Kontrolle ohne Bestrahlung.

**Tabelle 3 Quantifizierung von Malonaldialdehyd, Zellproteinen und GSH in Fibroblasten (Ergebnisse in % bezogen auf die Kontrolle, Mittelwert aus 2 Versuchen, jeder mit drei Wiederholungen**

| **Konzentration (Gew.%)** | **MDA-Spiegel** | **Gehalt an Zellproteinen** | **Anteil an GSH** |
|---|---|---|---|
| Kontrolle ohne UVA | 0 | 105 | 100 |
| UVA (20 J/cm²) | 100 | 100 | 49 |
| UVA + Extrakt nach Beispiel 1 0,003 % | 79 | 102 | 45 |
| UVA + Extrakt nach Beispiel 2 0,001 % | 42 | 117 | 83 |
| UVA + Extrakt nach Beispiel 3 0,003 % | 34 | 117 | 94 |

Die Ergebnisse aus der Tabelle 3 zeigen, dass die erfindungsgemäßen Extrakte aus den Blättern der Pflanze Argania spinosa signifikant den Grad an MDA in humanen Fibroblasten, weicher durch UVA-Strahlen induziert wird, reduzieren. Des weiteren ergibt sich eine hohe Aktivität, den Anteil an GSH in humanen Fibroblasten nach einer Bestrahlung mit UVA-Strahlung relativ konstant zu halten. Diese Ergebnisse zeigen eine hohe Kapazität von Extrakten aus den Blättern von Argania spinosa schädliche Effekte eines oxidativen Stresses auf der Haut zu reduzieren.

### 7. Beispiel: Entzündungshemmende Eigenschaften in vitro - UVB Lichtschutz

### Zellschutzwirkung gegen UVB an in vitro gezüchteten menschlichen Keratinozyten

Hintergrund: UVB-Strahlen (von 280 bis 320 nm) lösen durch Aktivierung eines Enzyms, nämlich Phospholipase A2 oder PLA2, die Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasmaenzyms LDH (Lactat Dehydrogenase) bestimmt wurde. Dieses Enzym dient als Marker für eine Zellschädigung.

Zur Durchführung der Tests wurde ein definiertes Medium (DMEM), das 10 % fötales Kälberserum enthält, mit den Keratinozyten beimpft und der Pflanzenextrakt (mit Saline-Lösung verdünnt) 72 Stunden nach dem Beimpfen zugegeben.

Die Keratinozyten wurden sodann mit einer UVB-Dosis bestrahlt (50 mJ/cm² - Röhren: DUKE GL40E).

Nach weiterer 1 tägiger Inkubation bei 37 °C und bei 5 % CO₂ wurde der LDH- und der PGE2-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche) Der Gehalt an PGE2 wurde mit einem ELISA-Test (ELISA Kit der Firma Roche) bestimmt. Nach der Trypsin-Behandlung wurden die Zellen zentrifugiert und ausgezählt.

**Tabelle 4: Zellschutzwirkung eines Extraktes von Blättern von Argania spinosa gegen UVB-Strahlen; Ergebnisse in % bezogen auf die Kontrolle, Mittelwert aus 2 Versuchen, jeder mit zwei Wiederholungen**

| **Extrakt nach Beispiel 1** | | **Gehalt an freigesetzte LDH (%)** |
|---|---|---|
| Kontrolle ohne UV | | Anzahl Keratinocyten (%) |
| Kontrolle mit UVB (30 mJ/cm2) | 49 | 100 |
| UVB + Extrakt 0,001 % | 73 | 11 |

Die Ergebnisse dieser Tests belegen, dass ein erfindungsgemäßer Extrakt der Pflanze Argania spinosa den Effekt von UVB-Strahlung auf die Anzahl an Keratinocyten reduziert. Es zeigt sich eine Verringerung des Gehalts an freigesetzte LDH im Cytoplasma. Die beschriebenen Extrakte zeigen demnach die Fähigkeit, die durch UVB-Strahlung hervorgerufene Schädigung an Zellmembranen zu reduzieren und zeigen eine hemmende Wirkung gegen Entzündungen, die durch UVB Strahlung induziert werden.

### 8: Beispiel: Inhibierung der Elastase-Aktivität.

Serin-Proteasen, wie z.B. Elastase oder Collagenase, bewirken den Abbau von Elastin, Proteoglycanen und Collagen und verursachen damit eine Schwächung des Bindegewebes. Im folgenden Test wurden die inhibierenden Eigenschaften der Extrakte gegenüber einer Pankreas-Elastase in zwei Systemen, nämlich einmal in einen chromogenen synthetischen Substrat A und zum anderen in einem natürlichen Substrat B (Elastin/Kongorot) untersucht. Die Einsatzmenge der Extrakte betrug 0,3 Gew.-%, die Inkubationszeit 30 min (20 °C). Die Inhibierung wurde photometrisch bei 410 bzw. 520 nm verfolgt, als Standard (= 0 % Inhibierung) diente α1-Antitrypsin. Die Ergebnisse sind in Tabelle 5 zusammengefaßt.

**Tabelle 5: Elastase-Inhibierung [%-absolut]**

| | **Substrat A** | **Substrat B** |
|---|---|---|
| α1-Antitrypsin | IC50 = 0,01 % | IC50 = 0,034 % |
| Extrakt nach Beispiel 1 | 51 % | 18% |
| Extrakt nach Beispiel 2 | 57% | 66% |
| Extrakt nach Beispiel 3 | 100% | 6% |

Der biochemische Test einer Collagenase Inhibierung wurde realisiert mit einer Collagenase aus Chlostridium histolyticum in einem chromogenen synthetischen Substrat C: FALGPA (furylacryloyl-Leu-Gly-Pro-Ala) einem spezifischen Substrat für Collagenase, welches nicht durch das Enzym hydrolysiert wird. Bezogen wurde dieses Substrat von SIGMA.

Die Einsatzmenge der Extrakte betrug 0,3 Gew.-%, die Inkubationszeit 30 min (20 °C). Die Inhibierung wurde untersucht durch Bestimmung der optischen Dichte "OD" bei 234 nm.

**Tabelle 6 : Collagenase-Inhibierung [%-absolut]**

| | **Substrat C** |
|---|---|
| Cystein | IC50 =1,56 % |
| Extrakt nach Beispiel 1 | 76% |
| Extrakt nach Beispiel 2 | 100% |

Der biochemische Test der Inhibierung von Plasmin, einer speziellen Serin-Protease wurde gegen den positiven Standard Aprotinine bestimmt. Für einen Extrakt nach Beispiel 2 konnte ein EC50-Wert von 2 µg/ml bestimmt werden. Damit reicht bereits eine Konzentration von 2 µg/ml des genannten Extraktes aus Blättern von Argania spinosa ume eine 50 %-ige Inhibierung der Enzymaktivität zu erreichen.

Die Extrakte aus den Blättern von Argania spinosa zeigen eine hohe Aktivität bei der Inhibierung der Proteasen Elastase und Collagenase.

### 9. Inhibierung der menschlichen MMP-1 Synthese

Untersucht wurde die Fähigkeit von Extrakten aus den Blättern von Argania spinosa den toxischen Einfluss von UV-A-Strahlen zu mindern. Dieser in vitro Test untersucht die Fähigkeit den Gehalt an Matrix-Metallo-Proteinasen wie beispielsweise MMP-1 zu reduzieren, die von menschlichen Fibroblasten nach UVA-Strahlung freigesetzt werden. Unter dem Einfluss von UVA-Strahlung werden vermehrt MMP freigesetzt. UVA-Strahlen wurden als Modell ausgewählt, da sie bis in die Dermis penetrieren , wo sie einen oxidativen Stress induzieren, welcher das Altern der Haut beschleunigt. Des weiteren ist es bereits bekannt, dass die Freisetzung von Matrix-Metallo-Proteinasen während des normalen Alterungsprozesses der Haut zunimmt. Eine Inhibierung der MMP würde also dem Alterungsprozess der Haut entgegenwirken.

Als in-vitro System diente eine Kultur dermaler Fibroblasten, bestimmt wurde die Freisetzung von MMP-1 aus diesen Fibroblasten unter dem Einfluss der UV-Strahlung.

Zur Versuchsdurchführung wurde eine Fibroblastenkultur in einem definiertem Kulturmedium (DMEM) mit fötalem Kalbsserum angesetzt und 2-3 Tage später mit den Testsubstanzen geimpft. Nach einer Inkubation von 24 h bei 37 °C und einem CO₂-Level von 5 Vol.-% wurde das Nährmedium durch eine Elektrolytlösung ersetzt und die Fibroblasten mit einer definierten UVA-Strahlungsmenge geschädigt (20 J/cm²). Nach dem Ende der Bestrahlung wurden die Fibroblastenkultur erneut 2 Tage inkubiert und anschliessend wurde an einer Probe des Überstandes der Kulturlösung der Gehalt an MMP-1 und TIMP 1 bestimmt. Unter der Abkürzung TIMP versteht man einen natürlich vorkommenden Inhibitor der Metallo Proteinase unter der Bezeichnung "Tissue-Inhibitor of Matrix-Metallo-Proteinase".

Die Bestimmung der MMP und der TIMP erfolgte mit zwei unterschiedlichen Kits, die unter der Bezeichnung RPN2610 und RPN2611von der Firma Amersham im Handel erhältlich sind. Die Ergebnisse sind in Tabelle 7 zusammengefasst. Angegeben ist die Menge MMP-1 und TIMP-1 in ng/ml aus einer Testreihe mit Dreifachbestimmung.

**Tabelle 7 Bestimmung des Menge an MMP-1 und TIMP-1 in ng/ml**

| | **Konzentration** | **MMP=1 in ng/ml** | | **TIMP-1 in ng/ml** | |
|---|---|---|---|---|---|
| | | ohne UVA-Strahlung | UVA 20 J/cm² | ohne UVA-Strahlung | UVA 20 J/cm² |
| Kontrolle | | 49 | 199 | 50 | 28 |
| Dexamethasone | 0,1 µM | 2 | 7 | 39 | 19 |
| Extrakt nach Bsp.1 | 0,0006 % | 31 | 121 | 54 | 25 |
| Extrakt nach Bsp.1 | 0,003 % | 27 | 88 | 47 | 19 |
| Extrakt nach Bsp.1 | 0,006 % | 16 | 72 | 40 | 14 |

Die Extrakte zeigen, dass Extrakte aus den Blättern von Argania spinosa die spontane Freisetzung von MMP-1 durch humane Fibroblasten signifikant reduzieren. Des weiteren zeigen sie, dass die erfindungsgemäßen Extrakte die Freisetzung von MMP-1 bei UVA-Bestrahlung nachhaltig vermindern.

Eine Verminderung des Gehaltes an TIMP-1 kann für die erfindungsgemäßen Extrakte sowie für Dexamethasone gefunden werden, was sich jedoch darauf zurückführen lässt, dass der Gehalt an MMP-1 bereits durch den Einfluss der Extrakte bzw. durch Dexamethasone reduziert wurde.

Diese Extrakte zeigen große Fähigkeiten die natürlichen Effekte der Hautalterung oder die Hautalterung durch UV-Strahlung zu reduzieren.

### 10. Beispiel: Beeinflussung der Melanogenese

Hintergrund: Die hautaufhellende Aktivität wurde untersucht mit einem Inhibierungstest an Tyrosinase und einem Inhibierungstest an Melanin Synthese auf B16 Melanozyten. Die Tyrosinase ist das Schlüsselenzym der Synthese des Melanins in den Melanozyten der menschlichen Haut. Dieses Enzym katalysiert die ersten beiden Etappen der Umwandlung von Tyrosin in Melanin, das heisst, die Oxidation von Tyrosin zu L-DOPA (Dihydroxyphenylalanin) und dann in Dopachrom.

Methode: 1. Tyrosinase Inhibierung: L-DOPA wurde mit Tyrosinase und dem zu testenden Extrakt vermischt,. Die optische Dichte des Dopachrome wurde bei 475 nm untersucht. Anschließend wurde die Kinetik untersucht und die Konzentration für eine 50-% igen Inhibierung (EC50) bestimmt.

2. lnhibierung der Melanogenese auf B16 Melanozyten: Die B16 Melanozyten werden in einem definierten Medium (DMEM mit 10% fötalem Kälberserum) kultiviert und 3 Tage bei 37 °C und 5% CO2 inkubiert. Das Wachstumsmedium wurde ersetzt durch das definierte Medium ohne Kälberserum welches einen bestimmten Anteil der zu testenden Extrakte enthielt. Nach einer weiteren 3-tägigen Inkubation wurde der Anteil an intakten Zellen durch den Gehalt an Zellproteinen nach der Methode von Bradford bestimmt (Anal. Biochem. 72, 248-254, 1976) und der Anteil an gebildetem Melanin druch Untersuchung der optischen Dichte bei 475 nm nach der Methode, beschrieien von Ando et al. 17e IFSCC Congress - Yokohama, 2, 909-918, 1992.

Die Vergleichssubstanz war Hydrochinon.

Die Ergebnisse wurden ermittelt als Aktivitätsindex im Verhältnis von Anteil Proteine zu Gehalt an Melanin: je größer der Index, desto höher ist die Inhibierungsaktivität und desto geringer ist die Stimulierung der Melanogenese.

**Tabelle 8: Beeinflussung der Melanogenese**

| | **Tyrosinase in tubo** | **Melanin Synthese** | |
|---|---|---|---|
| | | Konzentration (Gew-%) | Index |
| Hydrochinon | EC50 = 0,025 % | 0,0003 | 2,21 |
| Extrakt nach Beispiel 3 | EC50= 0,077% | 0,01 | 0,4 |

Die Ergebnisse zeigen für Extrakte nach Beispiel 3 eine Stimulierung der Melaninsynthese. Daraus ergibt sich der Einsatz dieses Extraktes als Pigmentierungsmittel.

### 11. Wirkung auf die Überlebensaktivität humaner Fibroblasten

Für die Beurteilung der Zellaktivität gibt es wesentliche Marker, zu denen MTT, Proteine und Glutathion zählen.

Das Überleben wurde durch die folgenden Gehalte ausgewertet:
- Rate des metabolisierten MTT (Methyl Thiazolyl Tetrazolium); Die Aktivität der Mitochondrien wird über den MTT-Test bestimmt. MTT wird durch ein Enzym der Respirationskette, Succinatdehydrogenase, in Formazan reduziert (Denizot F, Lang R, Rapid colorimetric assay for cell growth and survival. J. Immunol. Methods, 89, 271-277, 1986).
- von Proteinen; Die Proteinkonzentration der Zellen wurde bestimmt nach Bradford (Bradford M.M. A rapid and sensitive method for the quantitation of microgram quantities of protein utilizing the principle of protein-dye binding. Anal. Biochem. (1977) vol 72, pp 248-254)
- von Glutathion (GSH), ein direkt von der Zelle erzeugtes Peptid, zur Bekämpfung von oxydativem Stress oder von verschiedenen Schmutzstoffen, wie zum Beispiel Schwermetalle. Seine Synthese erfordert ATP als Energiequelle. GSH wurde bestimmt nach Hissin (Hissin P.J., Hilf R. A fluorometric method for determination of oxidised and reduced Glutathione in tissues. Analytical Biochemistry (1977) vol 74, pp 214-226). Glutathion (GSH) ist ein Peptid, das von Zellen produziert wird, um die Zelle vor oxidativem Stress oder Schwermetallen wie beispielsweise Blei oder Quecksilber zu schützen. Die drei Aminosäuren die bei der reduzierten Form von GSH involviert sind, sind wiederum verbunden mit spezifischen cytoplasmatischen Enzymen, die ATP benötigen.

Die Steigerung des GSH-Levels hat einen positiven Einfluss auf die Aktivität der Glutathion-S-transferase, die ein entgiftendes Enzym darstellt.

Methode: Menschliche Fibroblaste wurden in ein Nährmedium (DMEM = Dulbecco Minimum Essential Medium von der Firma Life Technologie Sarl) mit 10 % fötalem Kälberserum (von der Firma Dutcher) geimpft und 24 Stunden lang bei 37° C in einer 5 %igen CO₂-Athmosphär inkubiert.

Das Medium wurde danach durch ein Sub-Optimum-Medium (ohne SVF) ersetzt, das verschiedene Extrakte in verschiedenen Konzentrationen (0,01; 0,03 und 0,1 Gew.-%) nach der Beschreibung der Erfindung enthielt.

Die Ergebnisse werden im Verhältnis zu einer extraktfreien Formulierung für Protein, MTT und GSH in das Verhältnis gestellt und in einem Prozentsatz im Verhältnis zum unbehandelten Kontrollmittel angegeben als Durchschnittswert +/- SEM (Fehlertyp des Durchschnitts) ausgedrückt.

**Tabelle 9: Zellüberlebens - Test - (Ergebnisse in % basierend auf der Kontrolle ohne Extrakt (Mittelwert von 2 Assays in dreifacher Ausführung)**

| | **Konzentration in Gew.%** | **MTT** | **Proteine** | **GSH / Proteine** |
|---|---|---|---|---|
| Kontrolle | 0 | 100 | 100 | 100 |
| Extrakt nach Beispiel 3 | 0,0001 | 91 | 102 | 102 |
| | 0,0003 | 71 | 92 | 114 |
| | 0,001 | 56 | 77 | 128 |

Die Tabelle gibt jeweils die Mitochondrienaktivität über die MTT, Proteingehalte und die GSH-Gehalte an, die nach drei Tagen für verschiedene Konzentrationen an Extrakten gemessen wurden. Ein Extrakt der Blätter aus der Pflanze Argania spinosa nach Beispiel 3 mit einer Konzentration von 0,01 Gew.% ist in der Lage den GSH-Gehalt in menschlichen Fibroblasten um 28 % zu erhöhen.

Diese Ergebnisse zeigen, dass die Extrakte aus Blättern von Argania spinosa Fähigkeiten zur Verbesserung des Metabolismus (Synthese von Glutathion) durch die menschlichen Fibroblaste aufweisen, was deutlich eine energiespendende, stimulierende und "Anti-Älterungs"- Aktivität dieser Extrakte angibt.

### 12. Beispielrezepturen kosmetischer Mittel mit Extrakten aus den Blättern der Pflanze Argania spinosa

Die gemäß Beispiel 1 bis 3 erhaltenen Extrakte wurden in den folgenden erfindungsgemäßen Rezepturen K1 bis K21 sowie 1 bis 40 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

Alle in der Tabelle 10-13 aufgeführten und verwendeten Substanzen mit registrieriem Warenzeichen ® sind Marken und Produkte der COGNIS Gruppe.

**Tabelle 10: Softcreme Rezepturen K1 bis K7**

| (Alle Angaben in Gew.-% bezogen auf das kosmetische Mittel) | | | | | | | | |
|---|---|---|---|---|---|---|---|---|
| INCl Bezeichnung | K1 | K2 | K3 | K4 | K5 | K6 | K7 | V1 |
| Glyceryl Stearate (and) Ceteareth-12120 (and) Cetearyl Alcohol (and) Cetyl Palmitate | 8,0 | 8,0 | 8,0 | 8,0 | 8,0 | 8,0 | 8,0 | 8,0 |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakt nach Beispiel 1 bis 3 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 11: Nachtcremerezepturen K8 bis K14**

| (Alle Angaben in Gew.-% bezogen auf das kosmetische Mittel) | | | | | | | | |
|---|---|---|---|---|---|---|---|---|
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryllsononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Extrakt nach Beispiel 1 bis 3 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 12: W/O Bodylotion Rezepturen K15 bis K21**

| (Alle Angaben in Gew.-% bezogen auf das kosmetische Mittel) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl lsononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄•7 H₂O | 1,0 | 1,0 | 1,0 | 1,0 | 1,0 | 1,0 | 1,0 | 1,0 |
| Extrakt nach Beispiel 1 bis 3 | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

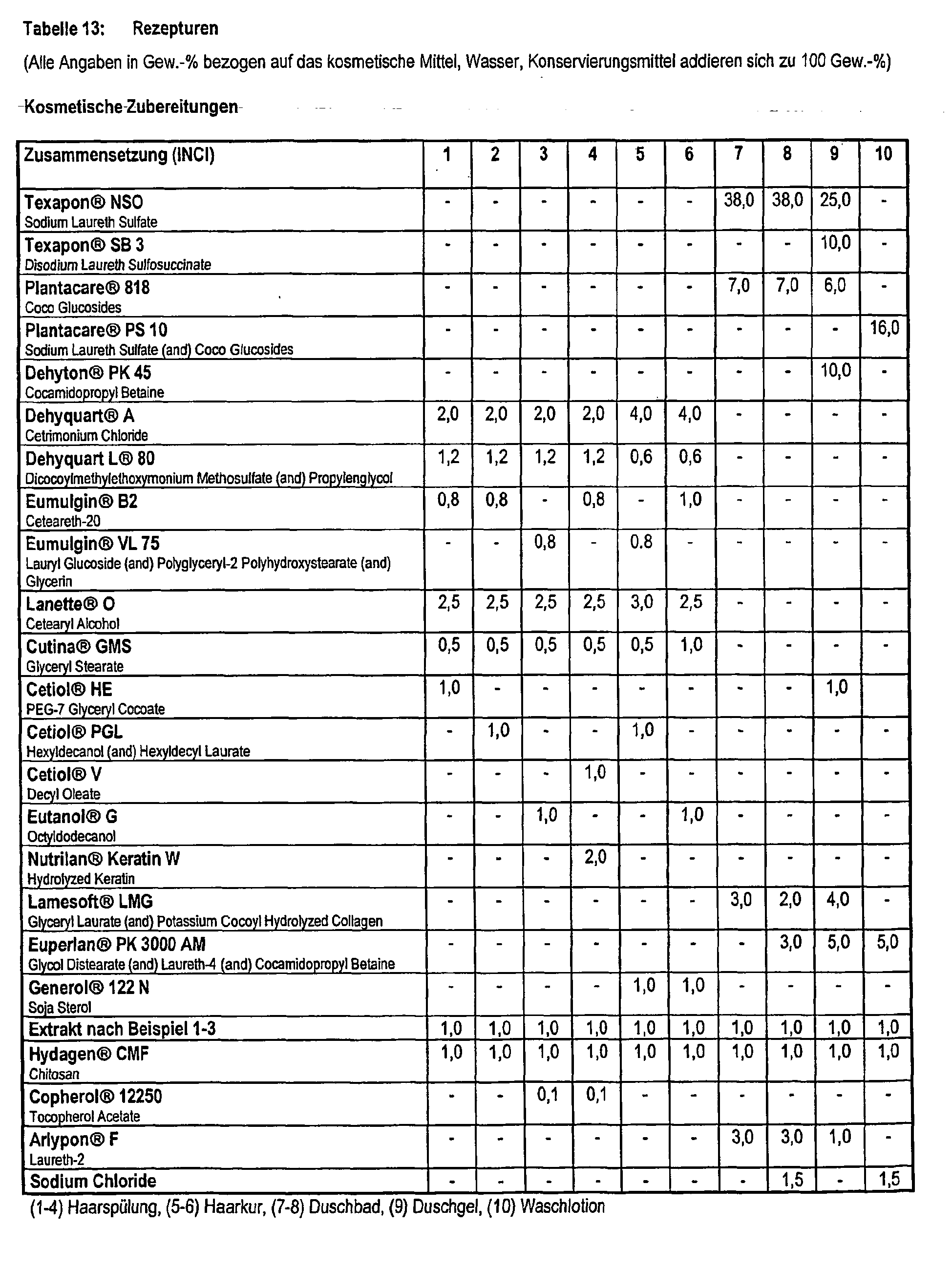

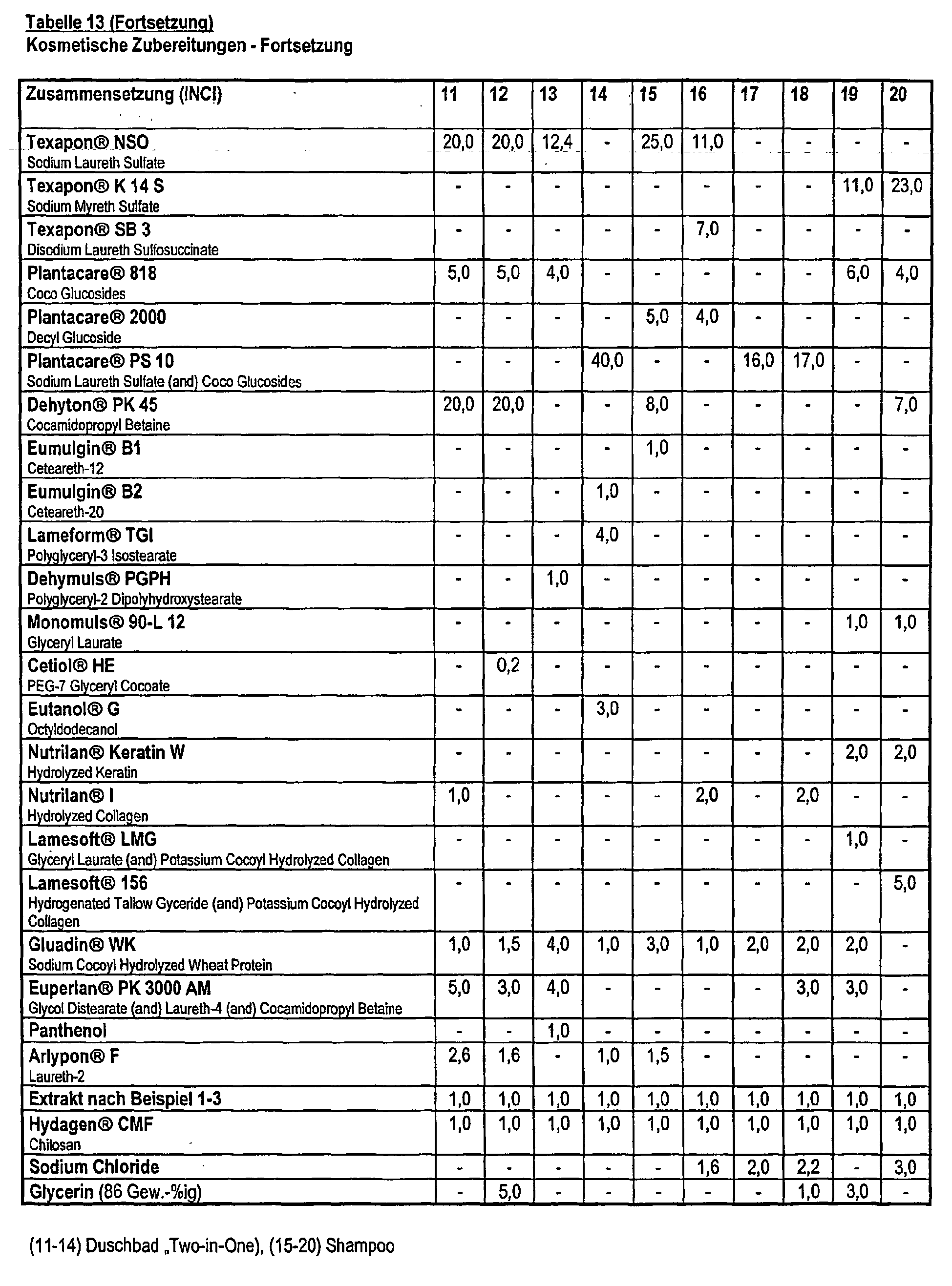

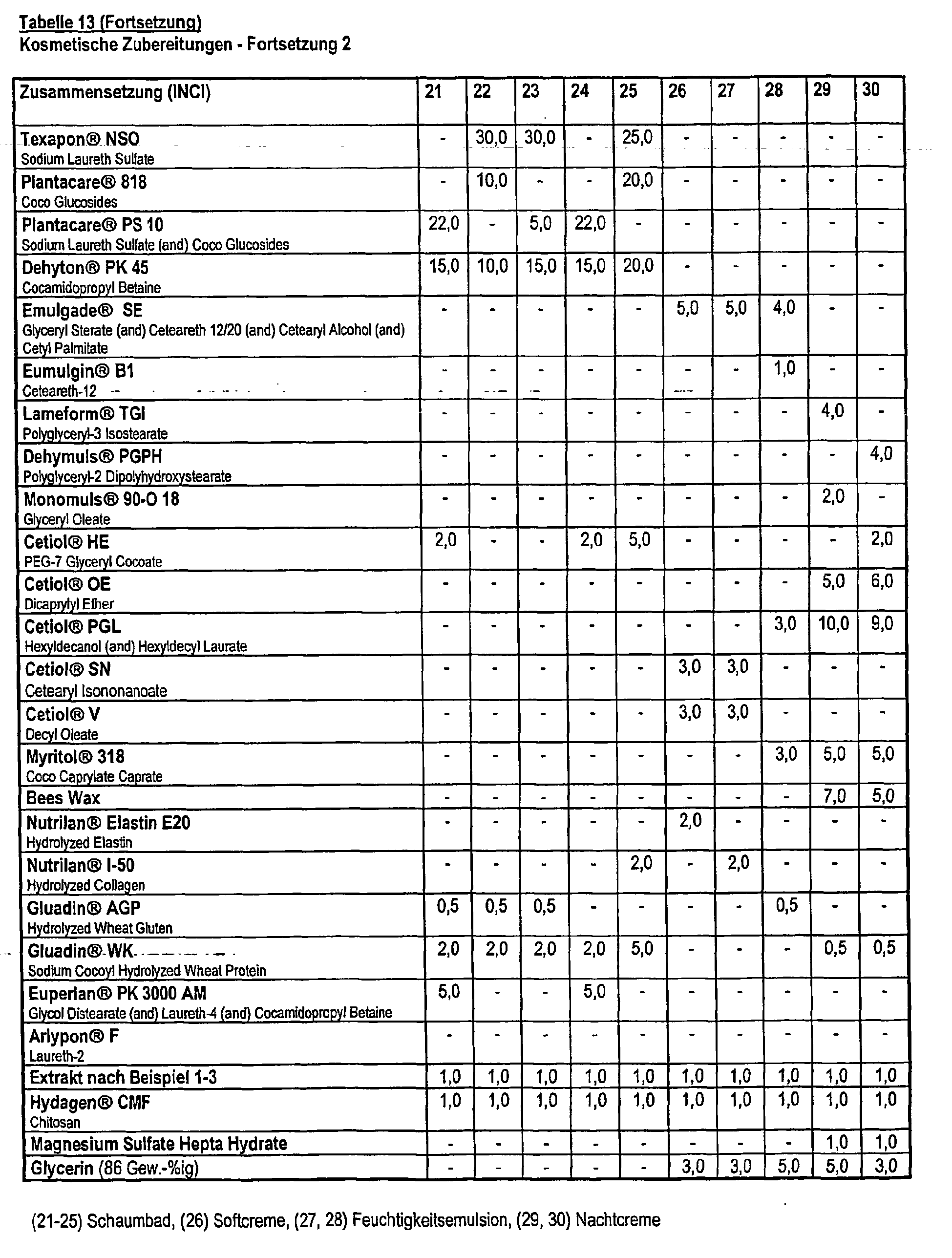

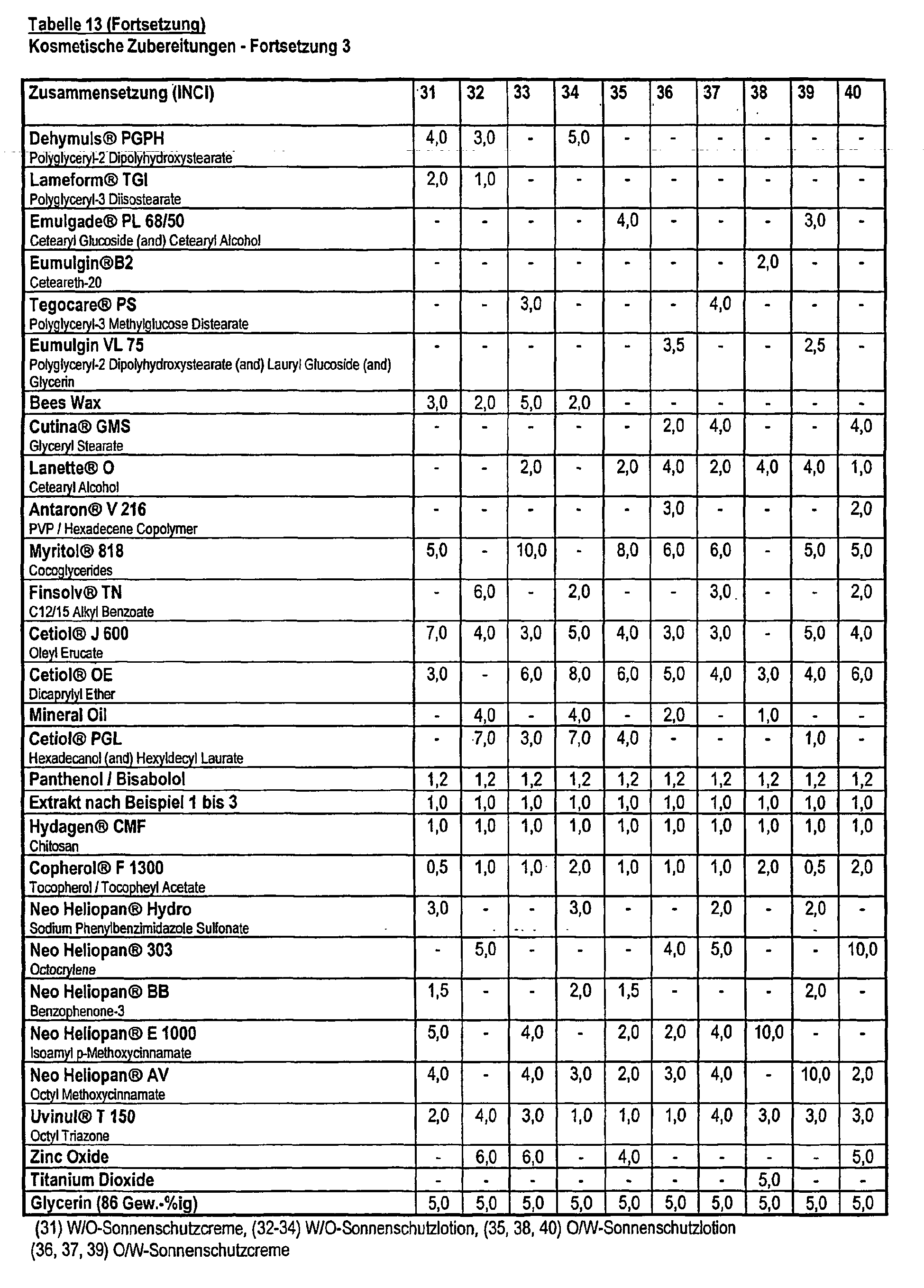

## Patentansprüche

1. Zubereitungen enthaltend Extrakte aus den Blättern der Pflanze Argania spinosa zur Verwendung als Medikament.

2. Zubereitungen nach Anspruch 1, **dadurch gekennzeichnet, dass** die Extrakte Substanzen enthalten, die ausgewählt sind aus der Gruppe, die gebildet wird von Flavonderivate, Saponoside, Oligomere Procyanolidine und Sterole.

3. Zubereitungen nach Anspruch 1 oder 2, **dadurch gekennzeichnet, dass** sie Flavonderivate enthalten, die ausgewählt sind aus der Gruppe, die gebildet wird von Myricetin-, Quercetin-, Gossypetin-, Kämpferol-, und Luteolin-glycosid, dass sie Oligomere Procyanolidine entbalten wie A2- Dimer und dass sie Sterole enthalten wie Spinasterol und/oder Scottenol.

4. Zubereitungen nach einem der Ansprüche 1 bis 3, **dadurch gekennzeichnet, dass** sie die Extrakte in Mengen von 0,01 bis 25 Gew.%, berechnet als Trockengewicht, bezogen auf die Zubereitung enthalten, mit der Maßgabe, dass sich die Mengenangaben mit Wasser und gegebenenfalls weiteren Hilfs- und Zusatzstoffen zu 100 Gew.% addieren.

5. Verwendung von Extrakten aus den Blättern der Pflanze Argania spinosa zur Herstellung eines Medikaments zum Sonnenschutz, insbesondere gegen UVA-**Strahlung und/oder gegen UVB-Strahlung.**

6. Verwendung von Extrakten aus den Blättern der Pflanze Argania spinosa zur Herstellung eines Medikaments, das als Antioxidans wirkt.

7. Verwendung von Extrakten aus den Blättern der Pflanze Argania spinosa zur Herstellung eines Medikaments mit anti-inflammatorischer Wirkung.

8. Verwendung von Extrakten aus den Blättern der Pflanze Argania spinosa zur Herstellung eines Medikaments, das antimikrobielle Wirkung hat.

9. Verwendung von Extrakten aus den Blättern der Pflanze Argania spinosa zur Herstellung eines Medikaments, das als protease-inhibierendes Mittel wirkt, insbesondere als MMP- und/oder Collagenase- und/oder Elastase-inhibierendes Mittel und bevorzugt als Plasmin Inhibitor.

10. Kosmetische Verwendung von Extrakten aus den Blättern der Pflanze Argania spinosa als Pflegemittel für Haut und/oder Haare.

11. Kosmetische Verwendung von Extrakten aus den Blättern der Pflanze Argania spinosa als Sonnenschutzmittel, insbesondere gegen UVA-Strahlung und/oder gegen UVB-Strahlung.

12. Kosmetische Verwendung von Extrakten aus den Blättern der Pflanze Argania spinosa als Antioxidans.

13. Kosmetische Verwendung von Extrakten aus den Blättern der Pflanze Argania spinosa gegen die Hautalterung.

14. Kosmetische Verwendung von Extrakten aus den Blättern der Pflanze Argania spinosa als protease-inhibierendes Mittel, insbesondere als MMP- und/oder Collagenase- und/oder Elastase-inhibierendes Mittel und bevorzugt als Plasmin Inhibitoren.

15. Kosmetische Verwendung von Extrakten aus den Blättern der Pflanze Argania spinosa als Pigmentierungsmittel.

## Claims

1. Preparation comprising leaf extracts of the plant Argania spinosa for use as a medicament.

2. Preparations according to Claim 1, **characterized in that** the extracts comprise substances chosen from the group formed by flavone derivatives, saponosides, oligomeric procyanolidines and sterols.

3. Preparations according to Claim 1 and/or 2, **characterized in that** they comprise flavone derivatives chosen from the group formed by myricetin glycoside, quercetin glycoside, gossypetin glycoside, kaempferol glycoside and luteolin glycoside, that they comprise oligomeric procyanolidines, such as A2 dimer, and that they comprise sterols, such as spinasterol and/or scottenol.

4. Preparations according to any of Claims 1 to 3, **characterized in that** they comprise the extracts in amounts of from 0.01 to 25% by weight, calculated as dry weight, based on the preparation, with the proviso that the quantitative data with water and optionally further auxiliaries and additives add up to 100% by weight.

5. Use of leaf extracts of the plant Argania spinosa in the manufacture of a medicament for sun protection, in particular against UVA radiation and/or against UVB radiation.

6. Use of leaf extracts of the plant Argania spinosa in the manufacture of a medicament which acts as antioxidant.

7. Use of leaf extracts of the plant Argania spinosa in the manufacture of a medicament having antiinflammatory action.

8. Use of leaf extracts of the plant Argania spinosa in the manufacture of a medicament which has antimicrobial action.

9. Use of leaf extracts of the plant Argania spinosa in the manufacture of a medicament which acts as protease-inhibiting agent, in particular as MMP- and/or collagenase- and/or elastase-inhibiting agent and preferably as plasmin inhibitor.

10. Cosmetic use of leaf extracts of the plant Argania spinosa as care agents for skin and/or hair.

11. Cosmetic use of leaf extracts of the plant Argania spinosa as sunscreens, in particular against UVA radiation and/or against UVB radiation.

12. Cosmetic use of leaf extracts of the plant Argania spinosa as antioxidant.

13. Cosmetic use of leaf extracts of the plant Argania spinosa against skin aging.

14. Cosmetic use of leaf extracts of the plant Argania spinosa as protease-inhibiting agent, in particular as MMP- and/or collagenase- and/or elastase-inhibiting agent and preferably as plasmin inhibitors.

15. Cosmetic use of leaf extracts of the plant Argania spinosa as pigmenting agents.

## Revendications

1. Préparations contenant des extraits issus des feuilles de la plante Argania spinosa pour une utilisation en tant que médicament.

2. Préparations selon la revendication 1, **caractérisées en ce que** les extraits contiennent des substances choisies parmi le groupe formé des dérivés de flavone, des saponosides, des procyanolidines oligomères et des stérols.

3. Préparations selon la revendication 1 ou 2, **caractérisées en ce qu'**elles contiennent des dérivés de flavone qui sont sélectionnés parmi le groupe formé du glycoside de myricétine, du glycoside de quercétine, du glycoside de gossypétine, du glycoside d'huile camphrée et du glycoside de lutéoline, **en ce qu'**elles contiennent des procyanolidines oligomères, telles qu'un dimère A2, et qu'elles contiennent des stérols, tels que le spinastérol et/ou le scotténol.

4. Préparations selon l'une des revendications 1 à 3, **caractérisées en ce qu'**elles contiennent les extraits dans des quantités comprises entre 0,01 et 25 % en masse, tel que calculé en tant que masse sèche, sur la base de la préparation, à la condition que les indications de quantité soient complétées avec de l'eau et éventuellement d'autres substances auxiliaires et additifs à 100 % en masse.

5. Utilisation d'extraits issus des feuilles de la plante Argania spinosa pour la production d'un médicament destiné à la protection solaire, en particulier contre le rayonnement UVA et/ou contre le rayonnement UVB.

6. Utilisation d'extraits issus des feuilles de la plante Argania spinosa pour la production d'un médicament qui agit en tant qu'antioxydant.

7. Utilisation d'extraits issus des feuilles de la plante Argania spinosa pour la production d'un médicament présentant une action anti-inflammatoire.

8. Utilisation d'extraits issus des feuilles de la plante Argania spinosa pour la production d'un médicament qui présente une action anti-microbienne.

9. Utilisation d'extraits issus des feuilles de la plante Argania spinosa pour la production d'un médicament qui agit en tant qu'agent inhibiteur de protéase, en particulier en tant qu'agent inhibiteur de MMP et/ou de collagénase et/ou en tant qu'agent inhibiteur d'élastase et, de préférence, en tant qu'inhibiteur de plasmine.

10. Utilisation cosmétique d'extraits issus des feuilles de la plante Argania spinosa en tant que produits de soins pour la peau et/ou les cheveux.

11. Utilisation cosmétique d'extraits issus des feuilles de la plante Argania spinosa en tant qu'agent de protection solaire, en particulier contre le rayonnement UVA et/ou le rayonnement UVB.

12. Utilisation cosmétique d'extraits issus des feuilles de la plante Argania spinosa en tant qu'antioxydant.

13. Utilisation cosmétique d'extraits issus des feuilles de la plante Argania spinosa contre le vieillissement cutané.

14. Utilisation cosmétique d'extraits issus des feuilles de la plante Argania spinosa en tant qu'agent inhibiteur de protéase, en particulier en tant qu'agent inhibiteur de MMP et/ou de collagénase et/ou en tant qu'agent inhibiteur d'élastase et, de préférence, en tant qu'inhibiteurs de plasmine.

15. Utilisation cosmétique d'extraits issus des feuilles de la plante Argania spinosa en tant qu'agent de pigmentation.
